# NEW EUROPEAN PATENT SPECIFICATION

(11) **EP 0 419 434 B2**
(45) Date of publication and mention of the opposition decision: **04.11.1998**
(45) Mention of the grant of the patent: 12.05.1993
(21) Application number: 90850312.1
(22) Date of filing: 20.09.1990
(51) Int. Cl.: A61F 13/15

(54) **A sanitary napkin or incontinence guard**
Hygiene-Binden oder Artikel gegen Inkontinenz
Serviette hygiénique ou article pour incontinent

(30) Priority: 20.09.1989 SE 8903090
(43) Date of publication of application: 27.03.1991
(73) Proprietor: Mölnlycke AB, 405 03 Göteborg (SE)
(72) Inventor: Lindquist, Bengt, S-443 51 Lerum (SE); Vastag, Eva, S-438 03 Härryda (SE)
(74) Representative: Romare, Laila Anette

(56) References cited:
- EP-A- 91 412
- EP-A- 389 023
- EP-A- 0 270 058
- EP-A- 0 304 644
- WO-A-88/04547
- DE-A- 3 515 804
- US-A- 2 064 431
- US-A- 2 331 355
- US-A- 2 747 575
- US-A- 3 912 565
- US-A- 4 490 147
- US-A- 4 552 795
- US-A- 4 848 572

## Description

The present invention relates to an absorbent article, such as a sanitary napkin or an incontinence guard, comprising a liquid-impermeable surface layer which is remote from the wearer when the article is worn, a liquid-permeable surface layer which faces towards the wearer when the article is worn, and an absorbent pad which is contained between the two surface layers. Such an absorbent article is disclosed in EP-A-0 304 644

Such articles are preferably narrow and are intended to be carried in the crotch part of a pair of underpants or panties, the article normally being secured to the panties by means of pressure-sensitive adhesive provided on the liquid-impermeable rear side of the article.

The primary drawback with absorbent articles of this kind is that they too often leak along the side edges of the article, therewith soiling the undergarment. The most common reason for leakage along the side edges of such an article is because during use the article will become deformed and fold or crumple in its long direction. It is not unusual for the side edges of the article and a part of its liquid-impermeable backing sheet to fold-in over its liquid-permeable front layer. Naturally, this will considerably reduce the liquid absorbency of the article, since only a narrow liquid-permeable part will remain between the inwardly folded edge parts.

It is known to reduce the risk of this type of lateral leakage resulting from deformation of the article in use, by forming with the aid of air-deposition techniques an absorbent pad which exhibits a raised part which is intended to lie against the genitals of the wearer when the article is in use. Secreted body fluid is sucked immediately into the article, since the article is in close abutment with the body of the wearer. Furthermore, the pre-shaped article will only be slightly deformed during use.

Although a pre-shaped article of this kind has been found to function satisfactorily with respect to leakage, it unfortunately has other drawbacks. The raised portion of the article is experienced by the wearer as being hard and uncomfortable and constitutes an obstacle or hinder when cycling, for instance.

SE 8605498-8 describes a sanitary napkin which has a soft, raised part on the side of the article that faces towards the wearer in use. This raised part is created by incorporating pre-tensioned elastification in the liquid-impermeable surface layer on the rear side of the napkin. Although a napkin of this kind is soft and comfortable to wear, it is not particularly attractive from an aesthetic aspect when worn, since the elastication draws or gathers together the liquid-impermeable layer of the napkin, such that even an unused napkin appears to be wrinkled and handled. Furthermore, it is almost impossible to provide adhesive fastener surfaces on the wrinkled undersurface of the napkin, and consequently it is necessary to support the napkin by means of some form of holder or girdle. This necessity is hardly acceptable to present-day users of such absorbent articles, however, since it has been customary for such users to simply secure the article directly to a pair of conventional underpants or panties.

US-A-2,331,355 discloses a catamenial pad which has a raised peak connecting two upwardly concave surfaces. The edges of this pad may be attached to an adhesive strip.

However, the present invention provides an absorbent article of the kind defined in the introduction which totally avoids the drawbacks of known articles of this kind. An article configured in accordance with the invention is given by the features of the second part of claim 1.

Further embodiments and features of the present invention are set forth in the following Claims.

The fact that the longitudinally extending side edges of an absorbent article are prevented from moving apart by more than a predetermined distance, as in accordance with the present invention, affords a number of advantages. For instance, it is impossible for the side edges of the article to fold in over the liquid-permeable surface layer of said article, since the side-edge restraining locking facility obtained with said article functions to urge the side edges to bend outwardly in the opposite direction. Furthermore, the side-edge restraining facility can be said to constitute a method of controlling the shaping and bending of the article while it is worn, so that, in use, the article will bend and flex so as to lie constantly against the body of the wearer. This enables secreted body liquid to be drawn directly into the absorbent pad and eliminates the risk of leakage past the side edges of the article. An article constructed in accordance with the invention contains essentially the same amount of absorbent material at its centre part as a conventional, flat absorbent article. This means that the article will be more responsive to the effect of external pressures than the earlier known napkins with which the raised portion is created by agglomorating absorbent material at the centre part of the napkin. This higher degree of compressibility of an inventive article means that the article will adapt more readily to the space between the thighs of the wearer. The article will therewith be soft and comfortable to the wearer and can be worn discretely.

An absorbent article having a soft arched portion and mutually locked side edges can be manufactured in a number of different ways. For instance, an arched absorbent body is produced by bending or arching a conventional, flat absorbent body. The maximum smallest distance between the opposing side edges of the absorbent pad is made permanent, either by making the liquid-impermeable layer on the rear side of the article smaller than the liquid-permeable layer on the front side of the article, or by applying separate locking tape which mutually connects the side edges of said article. This locking tape may, for instance, consist of shrink film which is extended transversely over the width of the article, suitably at its centre part.

Since the inventive absorbent article is produced from a flat absorbent pad which is subsequently arched, said pad may initially be straight or formed with a slightly wider centre part. In this latter case, the arched portion will contain more absorbent material. It is important, however, that the ultimate arched portion does not contain so much absorbent material as to detract from its softness and comfort in wear.

It has been found unsuitable to locate adhesive for securing the article to the wearer's underpants or panties immediately beneath the actual arched portion itself. Instead, the adhesive is preferably located along the end parts of the article, so that the article will conform to the contours of the wearer's body to the greatest possible extent.

The invention will now be described in more detail with reference to an exemplifying embodiment of an inventive absorbent article and with reference to the accompanying drawings.

Figure 1 of the drawings illustrates a sanitary napkin, seen from the side thereof which faces the wearer in use.

Figure 2 is a cross-sectional view of the sanitary napkin shown in Figure 1, taken on the line II-II.

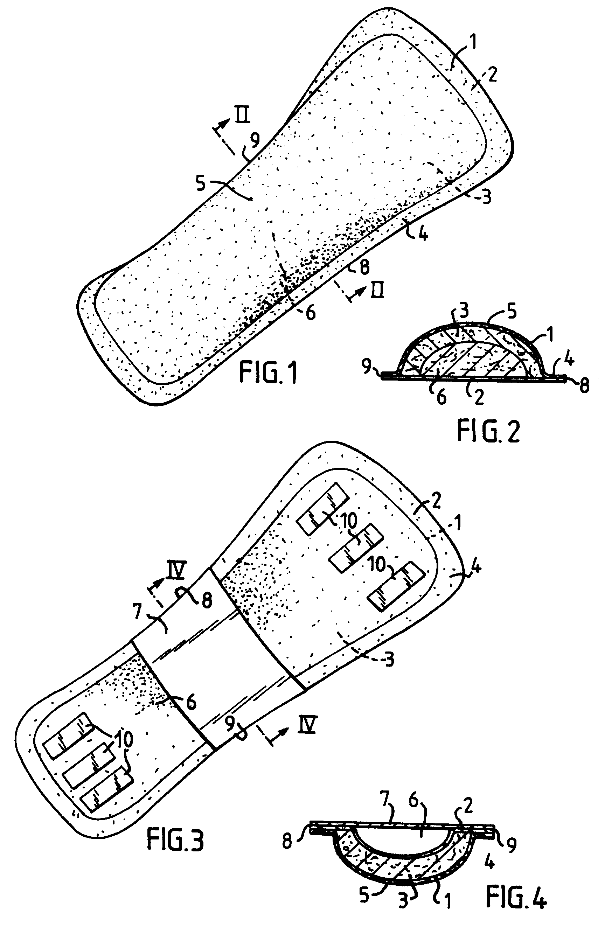

Figures 1 and 2 are shown with a filled cavity which are not part of the present invention and not covered by the claims, but which helps in understanding the invention.

Figure 3 illustrates a sanitary napkin, seen from the side which lies remote from the wearer in use.

Figure 4 is a sectional view of the sanitary napkin shown in Figure 3, taken on the line IV-IV.

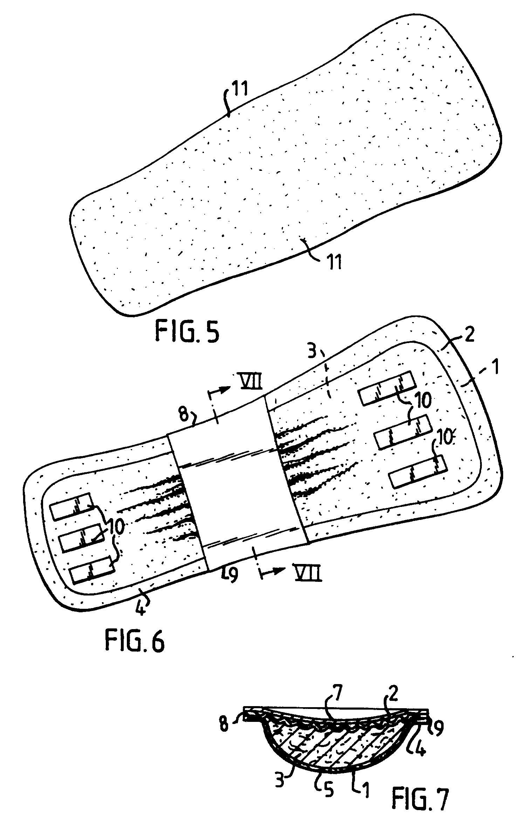

Figure 5 illustrates a flat absorbent pad intended for a sanitary napkin according to a further embodiment of the invention.

Figure 6 illustrates a still further embodiment of an inventive sanitary napkin, seen from the side of the napkin remote from the wear in use.

Figure 7 is a sectional view of the sanitary napkin shown in Figure 6, taken on the line VII-VII.

The sanitary napkin illustrated in Figures 1 and 2 includes a first liquid-permeable casing layer 1 on the side of the napkin which faces the wearer in use. The napkin also includes a liquid-impermeable second casing layer 2 on the side of the napkin remote from the wearer in use, and an absorbent pad or body 3, made for instance of cellulose fluff, which is contained between the two casing layers 1 and 2. The two casing layers 1, 2 extend slightly beyond the absorbent pad and the outwardly protruding casing parts 4 are mutually joined around the full circumference of the absorbent pad 3.

Because the liquid-impermeable casing layer 2 is narrower than the liquid-permeable casing layer 1, the absorbent pad 3 is held arched and presents a convex surface 5 on that side of the pad which faces towards the liquid-permeable outer layer 1. Located between the absorbent pad 3 and the liquid-impermeable casing layer 2 is a cavity 6 which is more or less well defined. The size of the cavity 6 depends on the extent to which the absorbent pad 3 is arched and also on the choice of absorbent material. Cellulose fluff is not springy and therefore has practically no intrinsic restoring capacity. Consequently, an abosrbent pad which is made of such material is unable to return to its original shape subsequent to being deformed. When no such constant deformation is desired, the absorbent pad may, of course, include a stiffening layer or elastic restoring means, for instance in the form of a plastic foam layer. The absorbent pad may also include so-called superabsorbents, these being materials capable of absorbing body fluid in quantities corresponding to several times their own weight. Examples of such materials are polyacrylates and modified cellulose. Superabsorbents may also be placed in the napkin cavity and there function to bind body fluid at a location remote from the body of the wearer.

The sanitary napkin illustrated in Figures 3 and 4 has essentially the same contruction as the sanitary napkin illustrated in Figures 1 and 2. In as far as Figures 3 and 4 relate to a tape of material which is not heat-shrinkable, the Figures 3 and 4 do not describe embodiments of the invention and are not covered by the claims. Consequently, similar napkin members have been identified with reference signs identical to those used in Figure 1. The casing layers 1 and 2 of this embodiment, however, have substantially the same geometric extension. In this case, the napkin is retained in its arched state with the aid of a restraining strip 7 attached to the centre part of the napkin, between the two side edges 8, 9 thereof. The restraining tape 7 may be attached to the liquid-impermeable casing layer 2 either by gluing or welding said tape. In order to achieve the desired napkin configuration, it has been found necessary for the restraining tape 7 to extend over at least 1/10 of the length of the absorbent pad 3 and preferably over at least 1/7 of said length.

The napkin embodiment illustrated in Figures 3 and 4 also exhibits pressure-sensitive adhesive surfaces at the two end parts of the liquid-impermeable casing layer 2. The adhesive surfaces 10 are intended to function as means for securing the napkin to a pair of underpants or like undergarment and said surfaces are preferably covered with protective tapes coated with a release agent, these protective tapes being removed before use. As before mentioned, an inventive napkin is preferably secured to the underpants of the wearer solely at the end parts of the napkin in use. This enables the napkin to conform effectively to the body of the wearer.

The absorbent body 3 illustrated in Figure 5 is flat and has a relatively broad centre part 11. Consequently, the centre part of the finished, arched absorbent pad will have a higher concentration of absorbent material, which can be beneficial, for instance, when the pad is used for an incontinence guard or in a sanitary napkin where menstruation is heavy and the pad must therefore be capable of absorbing a relatively large flow of liquid over a short time period.

The napkin illustrated in Figure 6 and 7 also has essentially the same construction as the earlier described napkins. The napkin according to Figure 6 and 7, however, distinguishes from the earlier described napkins in that it includes a restraining tape 7 of heat-shrinkable material. The restraining tape 7 is attached to the liquid-impermeable casing layer 2 across the whole of its surface lying against said layer 2. The restraining tape 7 is fastened to a flat napkin and thereafter caused to shrink and therewith arch the napkin. Since the liquid-impermeable casing material 2 is not shrinkable, it will be drawn or gathered together behind the restraining tape.

As with the case of the napkin of the Figure 3 and 4 embodiment, it is important that the restraining tape 7 extends over at least 1/10 of the length of the absorbent pad, and preferably over more than 1/7 of said length. Naturally, the whole of the liquid-impermeable casing layer can be made of a shrinkable material. This will then obviate the need for an additional restraining tape.

The restraining tape may also be positioned between two casing layers, for instance a plastic film and a fabric layer. This improves fixation of the restraining tape. Furthermore, the fabric layer will enhance the friction acting between the napkin and the underpants of the wearer.

The aforedescribed exemplifying embodiments do not limit the scope of the invention, since further modifications are conceivable within the scope of the following Claims.

## Claims

1. An absorbent article, such as a sanitary napkin or an incontinence guard, comprising a liquid-impermeable surface layer (2) which is remote from the wearer when the article is worn, a liquid-permeable surface layer (1) which faces towards the wearer when the article is worn, and an absorbent pad (3) which is contained between the two surface layers, wherin the absorbent pad (3) is arched transversely to the long axis of the pad, at least at its centre part, therewith to present a convex surface (5) on the side of the pad which faces towards the wearer in use, and that means are provided for preventing the smallest distance between the side edges (8,9) of the pad (3) at said arched part from exceeding a given value which is smaller than the width of the liquid-permeable surface layer (1) in a flat state, **characterized** in that said means consists of a tape of heat shrinkable material or the impermeable casing being of heat-shrinkable material, which has been caused to shrink and therewith arch the napkin.

2. An article according to Claim 1, **characterized** in that, when in a shrunk state, the liquid-impermeable casing layer (2) is narrower than the liquid-permeable casing layer (1), at least at the centre part of said article; in that the two casing layers (1,2) extend beyond the outer confines of the absorbent pad (3) around the whole of its circumference; and in that the outwardly projecting parts (4) of respective casing layers are joined together.

3. An article according to Claim 1, **characterized** in that said strip (7) of shrinkable material, at the centre part of the article, is attached at least to the side edges (8,9) of the liquid-impermeable casing layer (2) and extends between said two side edges; and in that when in a shrunk state the material strip (7) is shorter than the distance between the side edges (8,9) of the article when said article is in a flat state, whereby the article exhibits a convex surface (5) on the side of said article which faces towards the wearer in use.

4. An article according to Claim 3, **characterized** in that the material strip (7) is attached to the liquid-impermeable casing layer (2) over the whole of that surface which forms an abutment surface between said layer (2) and the material strip (7).

5. An article according to Claim 3 or 4, **characterized** in that the material strip (7) extends over at least 1/10 of the length of the absorbent pad, and preferably over at least 1/7 of said length.

## Patentansprüche

1. Absorbierender Artikel, wie beispielsweise eine Damenbinde oder ein Inkontinenzschutz, mit einer flüssigkeitsundurchlässigen Deckschicht (2), die dann von der Trägerperson abgewandt ist, wenn der Artikel getragen wird, einer flüssigkeitsdurchlässigen Deckschicht (1), die dann auf die Trägerperson zuweist, wenn der Artikel getragen wird, und einem absorbierenden Kissen (3), das zwischen den beiden Deckschichten liegt, wobei das absorbierende Kissen (3) wenigstens in seinem Mittelteil quer zu seiner Längsachse gebogen ist, wodurch auf der Kissenseite, die beim Gebrauch zur Trägerperson gerichtet ist, eine konvexe Fläche (5) dargeboten ist, und daß Mittel vorhanden sind, die verhindern, daß der kleinste Abstand zwischen den Seitenkanten (8, 9) des Kissens (3) im gebogenen Teil einen vorgegebenen Wert, der kleiner ist als die Breite der flüssigkeitsdurchlässigen Deckschicht (1) in flachem Zustand, überschreitet, **dadurch gekennzeichnet, daß** das Mittel aus einem Band aus wärmeschrumpfbarem Material besteht oder die undurchlässige Hülle aus wärmeschrumpfbaren Material besteht, das geschrumpft wurde und hiermit die Binde biegt.

2. Artikel nach Anspruch 1, **dadurch gekennzeichnet, daß** in einem Schrumpfzustand die flüssigkeitsundurchlässige Deckschicht (2) wenigstens im Mittelteil des Artikels schmaler ist als die flüssigkeitsdurchlässige Deckschicht (1), daß die beiden Deckschichten (1, 2) um den gesamten Umfang über die Außenbegrenzungen des absorbierenden Kissens (3) hinausreichen, und daß die nach außen vorstehenden Teile (4) der jeweiligen Deckschichten miteinander verbunden sind.

3. Artikel nach Anspruch 1, **dadurch gekennzeichnet, daß** der Streifen (7) aus schrumpfbarem Material im Mittelteil des Artikels an zumindest den Seitenkanten (8, 9) der flüssigkeitsundurchlässigen Deckschicht (2) angebracht ist und sich zwischen den zwei Seitenkanten erstreckt, und daß der Materialstreifen (7) in einem geschrumpften Zustand kürzer ist als der Abstand zwischen den Seitenkanten (8, 9) des Artikels, wenn der Artikel in einem flachen Zustand vorliegt, wobei der Artikel auf derjenigen Seite des Artikels, die beim Gebrauch zur Trägerperson hin gerichtet ist, eine konvexe Fläche (5) darbietet.

4. Artikel nach Anspruch 3, **dadurch gekennzeichnet, daß** der Materialstreifen (7) über die gesamte Fläche, die eine Anlagefläche zwischen der Schicht (2) und dem Materialstreifen (7) bildet, an der flüssigkeitsundurchlässigen Deckschicht (2) angebracht ist.

5. Artikel nach Anspruch 3 oder 4, **dadurch gekennzeichnet, daß** der Materialstreifen (7) sich über zumindest 1/10 der Länge des absorbierenden Kissens, und vorzugsweise über zumindest 1/7 dieser Länge, erstreckt.

## Revendications

1. Article absorbant, tel qu'une serviette hygiénique ou une protection contre l'incontinence, comprenant une couche superficielle (2) qui est imperméable aux liquides et qui se trouve à distance de l'utilisateur lorsque l'article est en service, une couche superficielle (1) qui est perméable aux liquides et qui se trouve vers l'utilisateur lorsque l'article est en service, et un tampon absorbant (3) qui est contenu entre les deux couches superficielles, article dans lequel le tampon absorbant (3) est courbé dans le sens transversal par rapport à son axe longitudinal, au moins dans sa partie centrale, de manière à présenter une surface convexe (5) sur le côté du tampon qui se trouve vers l'utilisateur pendant l'utilisation, et en ce que des moyens sont prévus pour empêcher que la distance la plus courte entre les bords latéraux (8, 9) du tampon (3) à l'endroit de ladite partie courbée dépasse une valeur donnée qui est plus petite que la largeur que présente la couche superficielle (1) perméable aux liquides dans un état plat. caractérisé en ce que lesdits moyens consistent en un ruban d'un matériau thermo-contractable ou la couche d'enveloppement imperméable étant en un matériau thermo-contractable qui a été amené à se contracter et qui, de ce fait, courbe la serviette hygiènique

2. Article selon la revendication 1, caractérisé en ce que, lorsqu'elle se trouve dans un état contracté, la couche d'enveloppement (2) imperméable aux liquides est plus étroite que la couche d'enveloppement (1) perméable aux liquides, au moins dans la partie centrale dudit article ; en ce que les deux couches d'enveloppement (1, 2) s'étendent au-delà des limites extérieures du tampon absorbant (3) sur la totalité de sa circonférence ; et en ce que les parties (4) qui font saillie vers l'extérieur et que comportent les couches d'enveloppement respectives sont assemblées l'une à l'autre.

3. Article selon la revendication 1, caractérisé en ce qu'il comprend ladite bande (7) de matériau contractable, dans la partie centrale de l'article, est fixée au moins aux bords latéraux (8, 9) de la couche d'enveloppement (2) imperméable aux liquides et s'étend entre les deux bords latéraux précités; et en ce que, lorsqu'elle se trouve dans un état contracté, la bande (7) de matériau est plus courte que la distance séparant les bords latéraux (8, 9) de l'article lorsque ledit article se trouve dans un état plat, grâce à quoi l'article présente une surface convexe (5) sur son côté se trouvant vers l'utilisateur pendant l'utilisation.

4. Article selon la revendication 3, caractérisé en ce que la bande (7) de matériau est fixée à la couche d'enveloppement (2), imperméable aux liquides, sur la totalité de la surface qui forme une surface d'appui entre ladite couche (2) et la bande de matériau (7).

5. Article selon la revendication 3 ou 4, caractérisé en ce que la bande de matériau (7) s'étend sur au moins 1/10 de la longueur du tampon absorbant, et de préférence sur au moins 1/7 de ladite longueur.
